# EUROPÄISCHE PATENTANMELDUNG

(11) **EP 0 655 450 A1**
(43) Veröffentlichungstag der Anmeldung: **31.05.1995**
(21) Anmeldenummer: 94116435.2
(22) Anmeldetag: 19.10.1994
(51) Int. Cl.: C07D 413/04, A61K 31/41, C07B 47/00

(54) **Verfahren zur Herstellung von Sydnoniminium-hydrogensulfaten sowie neue Zwischenprodukte**

(30) Priorität: 02.11.1993 DE 4337335
(71) Anmelder: CASSELLA Aktiengesellschaft, D-60386 Frankfurt (DE)
(72) Erfinder: Kujath, Eckard, Dr., D-64377 Maintal (DE); Schönafinger, Karl, Dr., D-63755 Alzenau (DE); Brendel, Joachim, Dr., D-61118 Bad Vilbel (DE)
(74) Vertreter: Muley, Ralf, Dr.

(57) **Zusammenfassung**

Die Erfindung betrifft ein Verfahren zur Herstellung von Sydnoniminium-hydrogensulfaten der allgemeinen Formel I, 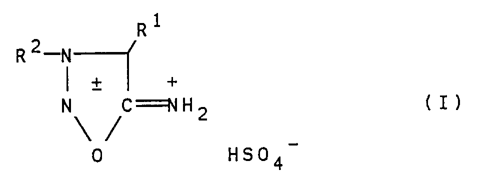
in der R¹ beispielsweise Wasserstoff oder Alkyl und R² eine substituierte Aminogruppe bedeutet, durch Umsetzung von N-Nitrosoaminoacetonitrilen mit Schwefelsäure. Die Sydnoniminium-hydrogensulfate finden insbesondere als Zwischenprodukte bei der Herstellung von N-Acylsydnoniminen oder anderen Sydnoniminium-Salzen Verwendung.

## Beschreibung

Die vorliegende Erfindung betrifft ein Verfahren zur Herstellung von Sydnoniminium-hydrogensulfaten und die Verwendung von Sydnoniminium-hydrogensulfaten als Zwischenprodukte für die Herstellung von Sydnonimin-Derivaten.

Es ist bekannt, daß 3-Aminosydnonimin-Derivate wertvolle pharmakologische Eigenschaften aufweisen. Beispielsweise werden N-Ethoxycarbonyl-3-morpholino-sydnonimin (Molsidomin) und 3-Morpholinosydnoniminium-chlorid (Linsidomin) als Herz-Kreislauf-Therapeutika eingesetzt. Weitere Sydnonimin-Derivate sind beispielsweise in der EP-A-406 659, EP-A-406 661, US-A-5 120 732, oder US-A-5 221 680 beschrieben.

Zur Synthese der Sydnonimin-Derivate geht man im allgemeinen von N-Nitrosoaminoacetonitrilen aus, die in Gegenwart von Säuren zu Sydnoniminen cyclisiert werden, die dabei in Form von Säureadditionssalzen anfallen. Das Säureadditionssalz des Sydnonimins wird dann gegebenenfalls in ein am exocyclischen Stickstoffatom acyliertes Sydnonimin oder in das Salz einer anderen als der für die Cyclisierung verwendeten Säure überführt, das für die vorgesehene Verwendung günstigere Eigenschaften hat. Als prinzipiell geeignete Säuren für die Cyclisierung der N-Nitrosoaminoacetonitrile zu den Sydnoniminen werden beispielsweise in der US-A-5 221 680 Chlorwasserstoff, Schwefel-, Salpeter-, Phosphor- oder Trifluoressigsäure oder Sulfonsäuren aufgeführt, bevorzugt ist aber Chlorwasserstoff, der in den konkret beschriebenen Cyclisierungen ausschließlich eingesetzt wird und zu Sydnoniminium-chloriden führt (siehe z.B. EP-A-406 659, EP-A-406 661, US-A-3 833 589, US-A-5 120 732, US-A-5 221 680, DE-A-1 670 127, Helvetica Chimica Acta, Band 45, S.2426 ff., (1962) oder Journal of Heterocyclic Chemistry, Band 7, S.123 ff., (1970)); daß konkret genannte N-Nitrosoaminoacetonitrile mit Schwefelsäure umgesetzt wurden, ist in keinem Fall beschrieben.

Die Cyclisierung von N-Nitrosoaminoacetonitrilen mit Chlorwasserstoff ist jedoch vielfach mit Nachteilen behaftet, da hierbei verschiedene Nebenreaktionen auftreten können, deren Ausmaß z.B. von den Substituenten im Molekül abhängt, und da die bei der Cyclisierung mit Chlorwasserstoff anfallenden Sydnoniminium-chloride oft verfahrenstechnisch ungünstige physikalisch-chemische Eigenschaften haben. Solche Nebenreaktionen sind z.B. die Denitrosierung unter Abspaltung von Nitrosylchlorid (siehe z.B. Russian Chemical Review (Uspekhi Khimii), Band 49, S.28 ff. (1980) oder Journal of Organic Chemistry of the USSR (Zhurnal Organicheskoi Khimii), Band 3, S.1470 ff., (1967)) oder die Bildung von Ammoniumchlorid, der eine Zersetzung des N-Nitrosoaminoacetonitrils zugrunde liegt. Diese Nebenreaktionen verringern die Ausbeute an gewünschtem Sydnonimin und führen zur Entstehung von Nebenprodukten, die die Kristallisation erschweren und häufig eine Reinigung des Produkts für die Verwendung in der Folgestufe erforderlich machen.

Die Löslichkeitseigenschaften der bei der Cyclisierung mit Chlorwasserstoff anfallenden Sydnoniminium-chloride bedingen teilweise aufwendige Aufarbeitungsverfahren oder den Einsatz spezieller Lösungsmittel, die einer Übertragung in den technischen Maßstab wegen ihrer Langwierigkeit, der benötigten Apparate und auftretender Sicherheitsprobleme entgegenstehen. So muß z.B. bei dem in der US-A-5 221 680 beschriebenen Verfahren zur Herstellung von 3-(cis-2,6-Dimethylpiperidino)sydnoniminium-chlorid wegen der hohen Löslichkeit dieses Salzes das Reaktionsgemisch eingeengt werden und dann der Rückstand mit Diethylether, dessen Einsatz im technischen Maßstab aufwendige Explosionsschutzmaßnahmen erfordern würde, verrührt werden, um das Produkt zur Kristallisation zu bringen. Ein Nacharbeiten der Vorschrift ergibt, daß das so erhaltene Produkt mit 5 % Ammoniumchlorid verunreinigt ist. Zur Reinigung ist in der US-A-5 221 680 eine Umkristallisation aus Acetonitril angegeben. Die Ausbeute am Sydnoniminium-chlorid, das Ausgangsmaterial für die Herstellung von N-Acylsydnoniminen oder Sydnoniminiumsalzen anderer Säuren ist, beträgt - bezogen auf eingesetztes 1-Amino-cis-2,6-dimethylpiperidin - nur 33 %. Ein weiterer Nachteil der technischen Synthese von Sydnonimin-Derivaten über die Sydnoniminium-chloride ist das beim Einsatz von Chlorwasserstoff auftretende Korrosionsproblem.

Aufgabe der vorliegenden Erfindung ist es, ein Verfahren bereitzustellen, das unter Vermeidung der genannten Nachteile, ohne aufwendige Aufarbeitungsmethoden und unter möglichst weitgehender Vermeidung der Bildung von Nebenprodukten, Sydnonimin-Derivate zugänglich macht.

Es wurde nun gefunden, daß diese Aufgabe gelöst wird, wenn N-Nitrosoaminoacetonitrile mit Schwefelsäure umgesetzt werden. Überraschenderweise sind dabei als ausschließliche Reaktionsprodukte die HydrogensulfatSalze der Sydnonimine erhältlich. Bei dieser bisher nicht bekannten Umsetzung fallen die entstehenden Sydnoniminium-hydrogensulfate direkt aus der Reaktionslösung in hoher Reinheit und hoher Ausbeute aus. Sie können gewünschtenfalls als solche als pharmakologische Wirkstoffe wie die entsprechenden Sydnoniminium-chloride eingesetzt werden, insbesondere dienen sie aber als Zwischenprodukte für die Herstellung von N-Acylsydnoniminen oder von anderen Säureadditionssalzen.

Gegenstand der vorliegenden Erfindung ist ein Verfahren zur Herstellung von Sydnoniminium-hydrogensulfaten der allgemeinen Formel I, 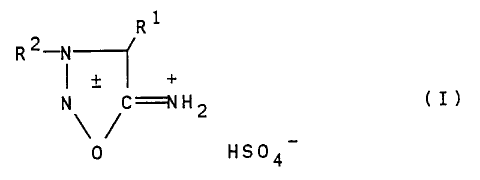
worin
R¹ Wasserstoff, Halogen, Alkyl, Cycloalkyl, Arylalkyl, Aryloxyalkyl, Arylthioalkyl, Alkoxyalkyl, Alkylthioalkyl oder Alkenylthioalkyl bedeutet;
R²- eine Aminogruppe der Formel 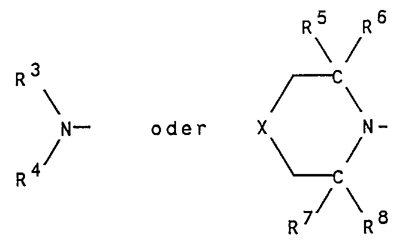
bedeutet;
R³ Alkyl, Alkenyl, Cycloalkyl, Bicycloalkyl, Tricycloalkyl, Alkoxyalkyl, Alkyl-S(O)ₙ-alkyl, Arylalkyl, das auch durch Alkyl oder Halogen substituiert sein kann, Phenyl, das auch durch Alkyl oder Halogen substituiert sein kann, Hydroxyalkyl, einen über eine Alkylenkette gebundenen heterocyclischen Ring oder einen Rest der Formel 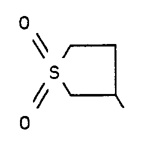
bedeutet;
R⁴ Wasserstoff bedeutet oder eine der Bedeutungen von R³ hat;
R⁵, R⁶, R⁷ und R⁸ unabhängig voneinander Wasserstoff oder Alkyl bedeuten;
X NR³, NSO₂R⁹, NCO₂Alkyl, S(O)ₙ, O, (CH₂)ₘ oder eine direkte Bindung bedeutet;
n 0, 1 oder 2 ist;
m 1, 2 oder 3 ist; und
R⁹ Alkyl, Aryl, Alkylaryl, Halogenaryl oder Dialkylamino bedeutet,
durch Umsetzung von N-Nitrosoaminoacetonitrilen der allgemeinen Formel II, 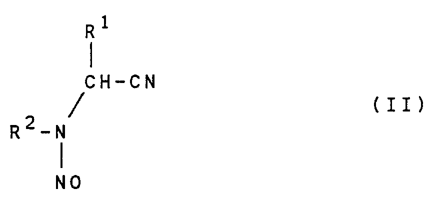
in der R¹ und R² die angegebenen Bedeutungen haben, mit Schwefelsäure in Gegenwart eines Lösungs- oder Dispergiermittels.

Alkylreste und Alkenylreste können geradkettig oder verzweigt sein und haben bevorzugt 1 bis 6 C-Atome, besonders bevorzugt 1 bis 4 C-Atome, im Falle der Alkylreste bzw. bevorzugt 3 bis 6 C-Atome im Falle der Alkenylreste. Dies gilt auch, wenn sie in Verbindung mit anderen Gruppen, z. B. als Alkoxy, Arylalkyl usw. vorliegen.

Beispiele für Alkylreste sind Methyl, Ethyl, Propyl, Isopropyl, Butyl, Isobutyl, sek.-Butyl und tert.-Butyl, Beispiele für Alkenylreste Allyl und 3,3-Dimethylallyl.

Cycloalkyl hat bevorzugt 5 bis 7 C-Atome und bedeutet besonders bevorzugt Cyclopentyl und Cyclohexyl.

Bicycloalkyl hat bevorzugt 7 bis 14 C-Atome. Tricycloalkyl hat bevorzugt 7 bis 16 C-Atome. Halogen bedeutet bevorzugt Fluor, Chlor, Brom oder Iod.

Aryl hat bevorzugt 6 bis 10 C-Atome und bedeutet besonders bevorzugt α- oder ß-Naphthyl oder Phenyl. Arylalkyl ist bevorzugt Benzyl und Phenylethyl. Aryloxyalkyl ist bevorzugt Phenoxymethyl und Phenoxyethyl. Entsprechendes gilt für Arylthioalkyl.

Arylreste können mono-, di- oder trisubstituiert sein, wobei jedoch auch bei einer Trisubstitution nur maximal 2 Nitrogruppen vorhanden sein können, wie beispielsweise 2-Methyl-4,6-dinitrophenyl und 2-Chlor-6-methyl-4-nitro-phenyl. Als Halogensubstituenten für die Arylreste kommen z.B. Chlor und Bromatome in Betracht. Als Arylreste sind insbesondere zu nennen: Methylphenyl (= Tolyl), Nitrophenyl und Chlorophenyl, insbesondere 4-Nitrophenyl und 4-Chlorophenyl.

R¹ steht bevorzugt für Wasserstoff.

R⁵, R⁶, R⁷ und R⁸ stehen bevorzugt unabhängig voneinander für Wasserstoff oder Methyl. Besonders bevorzugt stehen zwei dieser vier Reste für Methyl und die anderen beiden für Wasserstoff.

X steht bevorzugt für S(O)ₙ, O, CH₂ oder eine direkte Bindung, besonders bevorzugt für S oder CH₂.

Beispiele für den Rest R² sind Piperidino, Pyrrolidino, Dimethylamino, Diethylamino, Diisopropylamino, tert.Butyl-methylamino, tert.Butyl-ethylamino, tert.Butyl-propylamino, tert.Butyl-butylamino, tert.Butyl-(2-hydroxyethyl)amino, Dibenzylamino, Dicyclohexylamino, Morpholino, N-Methylpiperazino, 2,2-Dimethylpiperidino, 3,3-Dimethylmorpholino, 3,3-Dimethylthiomorpholino, 3,3-Dimethyl-1,1-dioxothiomorpholino, 3,3-Dimethyl-1-oxo-thiomorpholino, cis-2,6-Dimethylpiperidino, 2,2,6,6-Tetramethylpiperidino, 2,2-Dimethyl-N-methylsulfonyl-piperazino, 2,2-Dimethyl-N-tosyl-piperazino, 2,2-Dimethyl-pyrrolidino, 2,5-Dimethylpyrrolidino, 2,2-Dimethyl-N-dimethylaminosulfonyl-piperazino, Ethylamino, (2-Hydroxyethyl)amino und Cyclohexylamino.

Bevorzugt werden nach dem erfindungsgemäßen Verfahren Sydnoniminium-hydrogensulfate der allgemeinen Formel I hergestellt, in der R¹ für Wasserstoff und R²- für eine Aminogruppe der Formel 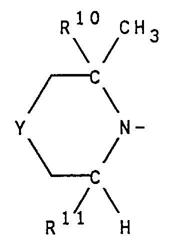
steht, in der
Y CH₂, O, S(O)ₙ oder eine direkte Bindung bedeutet,
n 0, 1 oder 2 ist und einer der Reste
R¹⁰ und R¹¹ eine Methylgruppe und der andere ein Wasserstoffatom bedeutet.

Besonders bevorzugt ist es, nach dem erfindungsgemäßen Verfahren zum einen das Sydnoniminium-hydrogensulfat der allgemeinen Formel I herzustellen, in dem R¹ für Wasserstoff und R² für den cis-2,6-Dimethylpiperidino-Rest steht, zum anderen dasjenige, in dem R¹ für Wasserstoff und R² für den 3,3-Dimethylthiomorpholino-Rest steht.

Das erfindungsgemäße Verfahren kann durchgeführt werden, indem über die gewünschte Zeitspanne Schwefelsäure zum N-Nitrosoaminoacetonitril der allgemeinen Formel II zudosiert oder die Verbindung der allgemeinen Formel II zu Schwefelsäure zudosiert wird oder indem beide Reaktionspartner gleichzeitig in das Reaktionsgefäß dosiert werden, wobei zudosierte und vorgelegte Reaktionspartner unabhängig voneinander in einem geeigneten Verdünnungsmittel gelöst oder dispergiert sein können. Das N-Nitrosoaminoacetonitril der allgemeinen Formel II kann in Substanz, sowohl in gereinigter Form als auch als Rohprodukt und sowohl trocken als auch lösemittelfeucht, in die erfindungsgemäße Umsetzung eingesetzt werden, es kann aber auch eine bei der vorangegangenen Herstellung des N-Nitrosoaminoacetonitrils erhaltene Lösung oder Suspension direkt eingesetzt werden und in Gegenwart des aus der vorangegangenen Reaktionsstufe stammenden Lösungs- oder Dispergiermittels, gegebenenfalls nach Zusatz weiterer Lösungsmittel, die Umsetzung mit Schwefelsäure erfolgen.

Die N-Nitrosoaminoacetonitrile der allgemeinen Formel II sind in den genannten Veröffentlichungen beschrieben. Ihre Herstellung erfolgt im allgemeinen durch Nitrosierung der entsprechenden Aminoacetonitrile, beispielsweise durch Einwirkung eines Alkalinitrits in Gegenwart einer Säure; die Aminoacetonitrile wiederum sind aus organischen Hydrazinen mit Aldehyden der Formel R¹CHO und Cyanid bzw. Blausäure erhältlich (siehe z.B. US-A-5 221 680, US-A-5 120 732).

Die Auswahl des Lösungs- oder Dispergiermittels hängt vom jeweiligen Einzelfall ab. Beispielsweise kann es, wenn für eine Folgeumsetzung eine Lösung des erfindungsgemäß hergestellten Sydnoniminium-hydrogensulfats benötigt wird, auch zweckmäßg sein, die Umsetzung des betreffenden N-Nitrosoaminoacetonitrils mit Schwefelsäure in einem Lösungsmittel durchzuführen, in dem das Produkt gelöst bleibt. Als Lösungs- oder Dispergier- bzw. Verdünnungsmittel kommen Kohlenwasserstoffe, halogenierte Kohlenwasserstoffe, Ketone, Ether, Ester, Alkohole, Sulfone, Sulfoxide, Carbonsäuren oder Wasser in Frage, wobei neben Einzelsubstanzen auch Gemische von zwei oder mehreren Substanzen als Lösungs- oder Dispergiermittel verwendet werden können. Als Beispiele seien genannt Hexan, Heptan, Kohlenwasserstoffgemische wie Benzine, Cyclohexan, Methylcyclohexan, Tetralin, Benzol, Toluol, Xylole, Ethylbenzol, Methylenchlorid, Chloroform, Chlorbenzol, Dichlorbenzol, Aceton, Methylethylketon, Diethylketon, Diisobutylketon, Diethylether, Di-n- und Di-iso-propylether, Dibutylether, Methyl-tert.Butylether, Tetrahydrofuran, 1,4-Dioxan, 1,2-Dimethoxyethan, Diethylenglykoldimethylether, Oligoethylenglykoldimethylether, Ameisensäuremethyl-, -ethyl- und -butylester, Essigsäuremethyl-, -ethyl-, -n-propyl-, -isopropyl-, -n-butyl-, -isobutyl- und -amylester, Propionsäuremethyl-, -ethyl- und -butylester, Methanol, Ethanol, n-Propanol, Isopropanol, Butanole, Pentanole, Ethylenglykol- und Diethylenglykolmonomethylether, Tetramethylensulfon, Dimethylsulfoxid, Ameisensäure, Essigsäure, Propionsäure. Als Lösungs- oder Dispergiermittel kann auch überschüssige Schwefelsäure verwendet werden bzw. fungieren.

Bevorzugt werden bei der erfindungsgemäßen Umsetzung Ether oder Carbonsäurealkylester als Lösungs- oder Dispergiermittel verwendet, insbesondere Methyl-tert.-butylether, 1,2-Dimethoxyethan, Diethylenglykoldimethylether, Tetrahydrofuran, Dioxan, Essigsäuremethyl-, -ethyl-, -n-propyl-, -isopropyl-, -n-butyl- oder -isobutylester oder Gemische hiervon, wobei in dem Lösungs- oder Dispergiermittel auch Wasser und übliche Nebenkomponenten enthalten sein können. Besonders bevorzugt ist die Durchführung in Essigsäureethylester. Dabei kann im Essigsäureethylester auch Wasser und beispielsweise Essigsäuremethylester, Methanol, Ethanol und Essigsäure enthalten sein.

Die Temperatur, bei der das erfindungsgemäße Verfahren zweckmäßig durchgeführt wird, hängt beispielsweise von der Reaktivität des N-Nitrosoaminoacetonitrils der allgemeinen Formel II, vom Lösungsmittel oder von der bei der Durchführung im technischen Maßstab vorgesehenen Reaktionsdauer ab. Im allgemeinen liegt die Temperatur bei -10°C bis 60°C. Bevorzugt wird die Reaktion bei 0°C bis 50°C, besonders bevorzugt bei 15°C bis 40°C, durchgeführt. Dabei kann es zweckmäßig sein, die Temperatur während der Durchführung der Umsetzung zu verändern, beispielsweise während des Zudosierens eines Reaktionspartners eine niedrigere und danach zur beschleunigten vollständigen Abreaktion eine höhere Temperatur einzustellen.

Bezogen auf 1 mol der zu cyclisierenden Verbindung der allgemeinen Formel II werden normalerweise 1 bis 3 mol Schwefelsäure eingesetzt, wobei ein Schwefelsäureüberschuß, wie bereits gesagt, zugleich als Lösungs- bzw. Dispergiermittel wirken kann. In bestimmten Fällen kann es aber auch zweckmäßig sein, unter Inkaufnahme eines Ausbeuteverlustes weniger als 1 mol Schwefelsäure pro Mol der Verbindung der allgemeinen Formel II einzusetzen, beispielsweise zwischen 0,8 und 1 oder zwischen 0,9 und 1 mol pro Mol der Verbindung der allgemeinen Formel II, z.B. wenn das Reaktionsgemisch mit dem entstandenen Sydnoniminium-hydrogensulfat direkt in eine Folgestufe eingesetzt werden soll, in der überschüssige Schwefelsäure stört. Bevorzugt werden pro Mol N-Nitrosoaminoacetonitril der allgemeinen Formel II 1 bis 2 mol Schwefelsäure eingesetzt, besonders bevorzugt 1,05 bis 1,5 mol, ganz besonders bevorzugt 1,1 bis 1,4 mol Schwefelsäure.

Die eingesetzte Schwefelsäure hat normalerweise einen Gehalt an H₂SO₄ von >90 Gew.%, bevorzugt von >95 Gew.%. Besonders bevorzugt ist der Einsatz von Schwefelsäure mit einem H₂SO₄-Gehalt von >98 Gew.%, darüber hinaus bevorzugt der der als Monohydrat bezeichneten ca. 100%igen Schwefelsäure. Als Nebenkomponenten können in der Schwefelsäure insbesondere Wasser und Polyschwefelsäuren bzw. Schwefeltrioxid enthalten sein.

Nach dem Zusammengeben der Reaktionspartner läßt man die Umsetzung des N-Nitrosoaminoacetonitrils der allgemeinen Formel II mit der Schwefelsäure bei der gewünschten Temperatur - gewünschtenfalls beispielsweise unter Rühren - ablaufen, bis der angestrebte Umsetzungsgrad erreicht ist. Die Umsetzung läßt sich im allgemeinen so steuern, daß schnell eine vollständige Umwandlung in das Sydnoniminium-hydrogensulfat erreicht ist. Durch die Löslichkeitseigenschaften der Sydnoniminium-hydrogensulfate gestaltet sich die Aufarbeitung vielfach besonders einfach, das direkt aus dem Reaktionsgemisch in kristalliner Form ausfallende Salz der allgemeinen Formel I kann - gegebenenfalls nach vorherigem Kühlen des Reaktionsgemischs -durch einfaches Filtrieren, Absaugen oder Zentrifugieren in hoher Ausbeute isoliert werden. So beträgt im Falle des 3-(cis-2,6-Dimethylpiperidino)sydnonimins die Ausbeute am Hydrogensulfat-Salz 86 % (bezogen auf eingesetztes 1-Amino-cis-2,6-dimethylpiperidin) (gegenüber nur 33 % Ausbeute am Chlorid-Salz bei Cyclisierung des N-(cis-2,6-Dimethylpiperidino)-N-nitrosoaminoacetonitrils mit Chlorwasserstoff nach dem bekannten Verfahren). In bestimmten Fällen kann es, wenn das Sydnoniminium-hydrogensulfat in Substanz isoliert und nicht das Reaktionsgemisch direkt in eine Folgestufe eingesetzt werden soll, je nach Substitutionsmuster und Lösungsmittel angebracht sein, das Reaktionsgemisch zur Erhöhung der Ausbeute zuerst nach bekannten Methoden im Vakuum oder bei Normaldruck einzuengen oder mit einem Fällungsmittel, in dem das Produkt besonders schwer löslich ist, zu versetzen oder anzuimpfen oder z.B. eine chromatographische oder extraktive Aufarbeitungsmethode anzuwenden.

Durch den glatten Verlauf des erfindungsgemäßen Verfahrens, bei dem konkurrierende Nebenreaktionen kaum zum Tragen kommen, werden die Sydnoniminium-hydrogensulfate der allgemeinen Formel I in hoher Reinheit und weitgehend frei von Nebenprodukten, wie beispielsweise Ammoniumsalzen, erhalten; die Produkte sind im Gegensatz zu den mit Chlorwasserstoff erhältlichen Sydnoniminium-chloriden nicht bräunlich, sondern weitgehend farblos. Im Falle des 3-(cis-2,6-Dimethylpiperidino)sydnonimins fällt das erfindungsgemäß hergestellte Hydrogensulfat-Salz mit einer durch HPLC ermittelten Reinheit von 99 % an, Ammonium ist nicht nachweisbar. Die Sydnoniminium-hydrogensulfate können normalerweise ohne weitere Reinigungsoperation weiterverwendet werden. Sollte in bestimmten Fällen, etwa bei direkter Verwendung als pharmakologischer Wirkstoff, eine weitere Reinigung notwendig sein, so kann sie nach den üblichen bekannten Methoden, insbesondere durch Umkristallisieren, erfolgen.

Die Sydnoniminium-hydrogensulfate der allgemeinen Formel I können direkt als pharmakologische Wirkstoffe eingesetzt werden, wie die entsprechenden Sydnoniminium-Salze mit anderen Anionen beispielsweise bei der Vorbeugung und Behandlung kardiovaskulärer Erkrankungen wie der Angina pectoris; insbesondere können sie aber als wertvolle Zwischenprodukte für die Herstellung anderer Sydnonimin-Derivate verwendet werden und beispielsweise in andere Sydnoniminium-Salze oder in N-Acyl- oder N-Sulfonylsydnonimine überführt werden, die noch bessere pharmakologische Wirkprofile oder günstigere Eigenschaften für die Herstellung galenischer Zubereitungen, z.B. eine höhere Stabilität, eine geringere Hygroskopizität oder einen vorteilhafteren pH-Wert, aufweisen; aufgrund der hohen Reinheit der erfindungsgemäß hergestellten Verbindungen der allgemeinen Formel I fallen auch die Folgeprodukte in besonders reiner Form an.

Die vorliegende Erfindung umfaßt daher auch Sydnoniminium-hydrogensulfate der oben angegebenen Formel I, in der R¹ und R² die oben angegebenen Bedeutungen haben, in der aber, wenn R¹ für Wasserstoff steht, R² nicht für den cis-2,6-Dimethylpiperidino-Rest stehen kann. Bevorzugte Sydnoniminium-hydrogensulfate der allgemeinen Formel I sind solche, in der R¹ für Wasserstoff und R²- für eine Aminogruppe der allgemeinen Formel 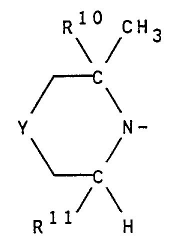
steht, in der Y CH₂, O, S(O)ₙ oder eine direkte Bindung bedeutet, n 0, 1 oder 2 ist und einer der Reste R¹⁰ und R¹¹ eine Methylgruppe und der andere ein Wasserstoffatom bedeutet, wobei aber, wenn Y CH₂ bedeutet, nicht R¹¹ eine zu der anderen Methylgruppe cis-ständige Methylgruppe bedeuten kann. Besonders bevorzugt ist das 3-(3,3-Dimethylthiomorpholino)sydnoniminium-hydrogensulfat.

Gegenstand der vorliegenden Erfindung ist weiterhin ein Verfahren zur Herstellung von N-Acylsydnoniminen der allgemeinen Formel III, 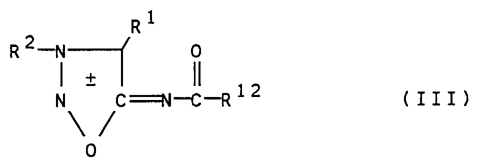
worin
R¹ und R² die oben angegebenen Bedeutungen haben und R¹² Aryl, durch 1 bis 3 Halogenatome und/oder 1 bis 3 Alkylreste und/oder 1 bis 3 Alkoxyreste und/oder 1 oder 2 Nitrogruppen mono-, di- oder trisubstituiertes Aryl, Arylalkenyl, Alkenylthioalkyl, Aryloxy, Alkoxycarbonyl, Pyridyl, Pyrrolyl, Imidazolyl, Pyrazolyl, Pyrazinyl, Pyrimidinyl, Pyridazinyl oder OR³ bedeutet oder eine der oben angegebenen Bedeutungen von R³ hat,
dadurch gekennzeichnet, daß ein Sydnoniminium-hydrogensulfat der allgemeinen Formel I mit einem Acylierungsmittel der allgemeinen Formel 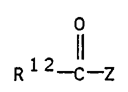
worin R¹² die angegebene Bedeutung hat und Z einen nucleophil abspaltbaren Rest bedeutet, umgesetzt wird.

Die obigen Erläuterungen zu den Alkyl-, Alkenyl-, Arylresten usw. und die oben erwähnten Beispiele gelten auch, wenn diese Reste für R¹² stehen oder in R¹² enthalten sind.

Z- steht z.B. insbesondere für Halogen, vorzugsweise -Cl oder -Br; -OH; -O-Alkyl, insbesondere mit 1 bis 5 C-Atomen; -O-Aryl, wobei der Arylrest insbesondere ein Phenylrest ist, der auch mit Alkyl, insbesondere Methyl, und/oder Nitro ein- oder mehrfach substituiert sein kann und beispielsweise ein Tolyl-, Dinitrophenyl- oder Nitrophenyl-Rest ist; -O-CO-R¹²; -O-CO-O-Alkyl, insbesondere mit 1 bis 5 C-Atomen im Alkylrest, oder den über ein N-Atom gebundenen Rest eines Azols oder Benzazols mit mindestens 2 N-Atomen im quasi-aromatischen Fünfring.

Die Acylierungsmittel stellen somit dar: für Z- = Halogen Säurehalogenide bzw. Halogenameisensäureester, von denen Säurechloride und Chlorameisensäureester bevorzugt sind; für -OH Carbonsäuren; für -O-Alkyl und -O-Aryl Ester, von denen die Tolyl-, 2,4-Dinitro- oder 4-Nitrophenylester bevorzugt sind; für -O-CO-R¹² Anhydride; für -O-CO-O-Alkyl gemischte Carbonsäure-Kohlensäure-Anhydride; oder heterocyclische Amide oder Azolide, insbesondere von Diazolen wie Imidazol. Die Acylierungsmittel können nach an sich bekannten Verfahren hergestellt werden.

Bei der Verwendung einer Carbonsäure als Acylierungsmittel ist der Zusatz eines Aktivierungsmittels zweckmäßig, das die Aufgabe hat, das Acylierungspotential der Carbonsäure zu erhöhen oder zu aktivieren bzw. die Carbonsäure in situ oder vorzugsweise kurz vor der Umsetzung mit der Verbindung der allgemeinen Formel I in ein reaktives Carbonsäurederivat zu überführen. Als derartige Aktivierungsmittel sind z.B. geeignet: N,N'-disubstituierte Carbodiimide, wie z.B. Dicyclohexylcarbodiimid; Kohlensäurederivate, wie z.B. Phosgen oder Chlorameisensäureester, insbesondere mit 1 bis 5 C-Atomen im Alkylrest; oder N,N'-Carbonyldiazole, wie z.B. N,N'-Carbonyldiimidazol. Als Aktivierungsmittel für Carbonsäuren sind auch z.B. Derivate der Oxalsäure, wie z.B. Oxalylchlorid, geeignet.

Bevorzugt ist die Herstellung von solchen N-Acylsydnoniminen der allgemeinen Formel III, in denen R¹ für Wasserstoff steht. Bevorzugt ist daneben weiterhin die Herstellung von Verbindungen der allgemeinen Formel III, in der R²- für eine Aminogruppe der Formel 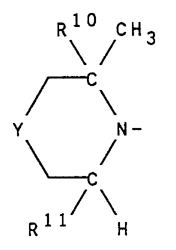
steht, in der Y CH₂, O, S(O)ₙ oder eine direkte Bindung bedeutet, n 0, 1 oder 2 ist und einer der Reste R¹⁰ und R¹¹ eine Methylgruppe und der andere ein Wasserstoffatom bedeutet. Besonders bevorzugt ist die Herstellung von N-Acyl-Derivaten aus 3-(cis-2,6-Dimethylpiperidino)sydnoniminium-hydrogensulfat sowie aus 3-(3,3-Dimethylthiomorpholino)sydnoniminium-hydrogensulfat.

R¹² steht bevorzugt für (C₁-C₄)-Alkyl, (C₅-C₇)-Cycloalkyl, (C₁-C₄)-Alkoxy-(C₁-C₄)-alkyl, (C₁-C₄)-Alkoxy, Phenyl oder einen durch 1 bis 3 Halogenatome und/oder 1 bis 3 (C₁-C₄)-Alkylreste und/oder 1 bis 3 (C₁-C₄)-Alkoxyreste mono-, di- oder trisubstituierten Phenylrest. Besonders bevorzugt steht R¹² für Methyl, Ethyl, Methoxy, Ethoxy, n-Butoxy, n-Butoxymethyl, Phenyl, 4-Chlorphenyl, 4-Methoxyphenyl oder 3,4-Dimethoxyphenyl, darüber hinaus bevorzugt für Ethoxy, Phenyl oder insbesondere 4-Methoxyphenyl.

Z- steht bevorzugt für -Cl, -O-CO-O-(C₁-C₄)-Alkyl oder -O-CO-R¹², besonders bevorzugt für -Cl.

Eine ganz besonders bevorzugte Ausführungsform des erfindungsgemäßen Verfahrens ist die, bei der R¹ Wasserstoff, R² den cis-2,6-Dimethylpiperidino-Rest, R¹² den 4-Methoxyphenyl-Rest und Z Chlor bedeutet.

Die Acylierung der Verbindungen der allgemeinen Formel I zu denen der allgemeinen Formel III wird in an sich bekannter Weise durchgeführt, zweckmäßigerweise in Gegenwart eines inerten Lösungs- oder Dispergier- bzw. Verdünnungsmittels, als das auch ein Überschuß des Acylierungsmittels fungieren kann, in flüssiger oder flüssiger disperser Phase, insbesondere auch in einem System mit zwei flüssigen Phasen, zweckmäßigerweise unter Rühren. Geeignete Lösungs- oder Dispergiermittel sind z.B. solche, die für die Durchführung der Umsetzung der Verbindungen der allgemeinen Formel II mit Schwefelsäure angegeben worden sind, darüber hinaus auch z.B. Pyridin und Amide wie Dimethylformamid. Auch Lösungsmittelgemische sind geeignet. Bevorzugt wird die Umsetzung in Wasser oder Essigsäureethylester, besonders bevorzugt in einem Gemisch aus Wasser und Essigsäureethylester, durchgeführt.

Die Acylierung kann prinzipiell bei Temperaturen zwischen -10^{O}C und dem Siedepunkt des verwendeten Lösungs-, Dispergier- oder Verdünnungsmittels erfolgen. In vielen Fällen wird die Umsetzung bei 0 bis 50^{O}C, insbesondere bei 0 bis 30^{O}C, durchgeführt.

Bei der Acylierung beträgt das Molverhältnis zwischen der Verbindung der allgemeinen Formel I und dem Acylierungsmittel theoretisch 1 : 1. Zweckmäßigerweise wird das Acylierungsmittel in einem geringen molaren Überschuß eingesetzt. Überschüsse von bis zu 30 mol% sind in der Regel ausreichend, d.h. das Molverhältnis zwischen der Verbindung der allgemeinen Formel I und dem Acylierungsmittel beträgt normalerweise 1 : (1 bis 1,3), vorzugsweise 1: (1 bis 1,2).

Normalerweise wird bei der Reaktion eine Base oder ein Basen-Gemisch als Säurefänger zugesetzt, z.B. ein Alkalihydroxid, wie z.B. Natrium-, Kalium- oder Lithiumhydroxid, ein tertiäres organisches Amin, wie z.B. Pyridin oder Triethylamin, ein Alkalicarbonat oder Alkalibicarbonat, wie z.B. Soda, Kaliumcarbonat oder Natriumbicarbonat, oder ein Alkalisalz einer schwachen organischen Säure, wie z.B. Natriumacetat. Bevorzugt werden Alkalimetallbicarbonate, besonders bevorzugt Natriumbicarbonat, verwandt. Bei der Acylierungsreaktion können auch geeignete Katalysatoren, wie z.B. 4-Dimethylaminopyridin, zugesetzt werden.

Die Isolierung der erhaltenen N-Acylsydnonimine der allgemeinen Formel III kann nach an sich bekannten Aufarbeitungsmethoden erfolgen, beispielsweise durch Filtrieren, Zentrifugieren, Extrahieren oder Chromatographieren oder Ausfällen, gegebenenfalls nach vorherigem Abkühlen oder Einengen anfallender Lösungen. Die erhaltenen N-Acylsydnonimine zeichnen sich durch eine besondere Reinheit aus. Gegebenenfalls können sie durch an sich bekannte Verfahren, insbesondere durch Umkristallisieren, noch weiter gereinigt werden.

Gegenstand der vorliegenden Erfindung ist auch ein Verfahren zur Herstellung von Sydnoniminium-Salzen der allgemeinen Formel IV, 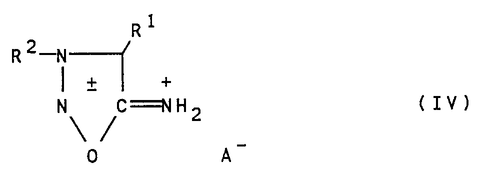
worin R¹ und R² die oben angegebenen Bedeutungen haben und A⁻ das Anion einer pharmakologisch annehmbaren ein- oder mehrbasigen anorganischen oder organischen Säure HA ist, die nicht Schwefelsäure ist, wobei aber, wenn R¹ für Wasserstoff und R² für den cis-2,6-Dimethylpiperidino-Rest steht, HA nicht für Phosphorsäure oder Weinsäure stehen kann, dadurch gekennzeichnet, daß ein Sydnoniminium-hydrogensulfat der allgemeinen Formel I mit der Säure HA und/oder einem Salz hiervon umgesetzt wird.

Geeignete Säuren HA, mit denen die Verbindungen der allgemeinen Formel I zu denen der allgemeinen Formel IV umgesetzt werden können, sind beispielsweise Chlorwasserstoff, Bromwasserstoff, Phosphor-, Salpeter-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Äpfel-, Sulfamin-, Phenylpropion-, Glucon-, Ascorbin-, Isonicotin-, Methansulfon-, p-Toluolsulfon-, Zitronen-, Adipinsäure oder Naphthalindisulfonsäuren, z.B. Naphthalindisulfonsäure(1,5). Bevorzugt werden Säuren eingesetzt, die zu Sydnoniminium-salzen der allgemeinen Formel IV führen, die besonders günstige physikalisch-chemische Eigenschaften für den Einsatz als pharmakologischer Wirkstoff aufweisen bzw. günstige Eigenschaften für die Herstellung galenischer Zubereitungen, z.B. eine gute Löslichkeit, eine hohe Stabilität, eine geringe Hygroskopizität oder einen vorteilhaften pH-Wert. Je nach vorgesehener Verwendungsart des Salzes der allgemeinen Formel IV und nach den Substituenten kann z.B. Phosphorsäure, Oxalsäure, Milchsäure, Weinsäure, Fumarsäure, Maleinsäure, Zitronensäure oder Methansulfonsäure besonders günstig sein.

Die Herstellung der Sydnoniminium-salze der allgemeinen Formel IV aus den Hydrogensulfaten der allgemeinen Formel I und den Säuren HA und/oder ihren Salzen kann wie üblich durch Vereinigung der Komponenten in einem geeigneten Lösungs- oder Dispergiermittel erfolgen. Geeignete Lösungs- oder Dispergiermittel sind beispielsweise Wasser, Alkohole, wie Methanol, Ethanol, Isopropanol, Butanol, Aceton, Acetonitril, Essigsäureethylester, Dimethylsulfoxid, Dimethylformamid oder Gemische dieser Lösungsmittel. Bevorzugte Lösungsmittel sind Wasser und Alkohole, wie Methanol, Ethanol und Isopropanol, oder die Gemische dieser Alkohole mit Wasser. Besonders bevorzugt ist Wasser.

Im allgemeinen wird bei der Herstellung der Verbindungen der allgemeinen Formel IV nach dem erfindungsgemäßen Verfahren eine Base oder ein Basen-Gemisch zugesetzt. Geeignete anorganische oder organische Basen sind beispielsweise Alkalimetallhydroxide, -carbonate und -hydrogencarbonate, wie Lithiumhydroxid, Natrium- und Kaliumhydroxid, Natriumcarbonat, Kaliumcarbonat, Natriumhydrogencarbonat oder Kaliumhydrogencarbonat, Salze schwacher Säuren, wie z.B. Natriumacetat, Ammoniak oder Amine, wie Ethylamin, Trimethylamin, Triethylamin, Ethanolamin oder Pyridin. Bevorzugte Basen sind Natriumhydrogencarbonat und Natron- und Kalilauge. Als Base kann insbesondere auch ein Salz der Säure HA eingesetzt werden, das vorab z.B. mit den genannten Basen aus der Säure HA erhalten wurde.

Der Austausch des Hydrogensulfat-Anions gegen das Anion der Säure HA wird normalerweise bei Temperaturen von -10°C bis 40°C, bevorzugt bei -5°C bis 30°C, besonders bevorzugt bei 0°C bis 25°C, durchgeführt.

Das Zusammengeben der Verbindungen der allgemeinen Formel I, der Säuren HA und/oder ihrer Salze und gegebenenfalls der Base erfolgt im allgemeinen unter Rühren. Die Komponenten können dabei als solche oder in Form von Lösungen zugesetzt werden. Die Reaktionsprodukte der allgemeinen Formel IV können durch an sich bekannte Aufarbeitungsverfahren isoliert werden. Im allgemeinen fallen sie direkt aus dem verwendeten Lösungs- oder Dispergiermittel aus und können dann in einfacher Weise, z.B. durch Filtrieren oder Zentrifugieren, gewonnen werden. Gewünschtenfalls können sie nach an sich bekannten Methoden, insbesondere durch Umkristallisation, weiter gereinigt werden.

### Beispiel 1:

### 3-(cis-2,6-Dimethylpiperidino)sydnoniminium-hydrogensulfat

Zu einer Lösung von 109 g 1-Amino-cis-2,6-dimethylpiperidin in 70 ml Wasser, die durch Zugabe von 37 gew.%iger Schwefelsäure auf pH 6,5 gestellt wurde, tropft man innerhalb von 3 h bei 20°C Lösungen von 50 g Natriumcyanid in 160 ml Wasser und 78 g 39gew.%igem Formaldehyd in 90 ml Wasser. Man hält den pH durch gelegentliche Zugabe von Schwefelsäure bzw. Sodalösung konstant, rührt über Nacht, stellt mit 37gew.%iger Schwefelsäure auf pH 3,5 und fügt 680 ml Essigsäureethylester zu. Dann tropft man eine Lösung von 64 g Natriumnitrit in 90 ml Wasser bei 20°C zu, rührt 2 h bei pH 3,5 und trennt die Phasen. Zu der organischen Phase, die das N-(cis-2,6-Dimethylpiperidino)-N-nitrosoaminoacetonitril enthält, tropft man bei 30°C 100 g Monohydrat in 30 min zu, rührt über Nacht und saugt den ausgefallenen Feststoff ab. Man wäscht mit Essigsäureethylester nach, trocknet im Vakuum und erhält 215 g (86 %) 3-(cis-2,6-Dimethylpiperidino)sydnoniminium-hydrogensulfat.
Fp. 157 bis 158°C (Zers.), Gehalt (HPLC): 99 %, kein Ammonium nachweisbar.

### Beispiel 2:

### 3-(cis-2,6-Dimethylpiperidino)sydnoniminium-hydrogensulfat

Zu einer Lösung von 19,6 g N-(2,6-cis-Dimethylpiperidino)-N-nitrosoaminoacetonitril in 80 ml 1,2-Dimethoxyethan tropft man bei 25 bis 30°C 11,85 g Monohydrat zu. Nach Rühren über Nacht kühlt man auf 0°C ab, saugt den ausgefallenen Niederschlag ab und erhält nach Trocknen im Vakuum 27,1 g (92 %) Produkt.

### Beispiel 3:

### 3-(cis-2,6-Dimethylpiperidino)sydnoniminium-hydrogensulfat

Analog dem Beispiel 2 erhält man aus 19,6 g N-(2,6-cis-Dimethylpiperidino)-N-nitrosoaminoacetonitril, 11,85 g Monohydrat und 280 ml Essigsäure-n-butylester 27,8 g (95 %) Produkt.

### Beispiel 4:

### 3-(cis-2,6-Dimethylpiperidino)sydnoniminium-hydrogensulfat

Analog dem Beispiel 2 erhält man aus 19,6 g N-(2,6-cis-Dimethylpiperidino)-N-nitrosoaminoacetonitril, 11,85 g Monohydrat und 80 ml Tetrahydrofuran 23,3 g (79 %) Produkt.

### Beispiel 5:

### 3-(cis-2,6-Dimethylpiperidino)sydnoniminium-hydrogensulfat

Analog dem Beispiel 2 erhält man aus 19,6 g N-(2,6-cis-Dimethylpiperidino)-N-nitrosoaminoacetonitril, 11,85 g Monohydrat und 100 ml Diethylenglykoldimethylether 26,4 g (90 %) Produkt.

### Beispiel 6:

### 3-(3,3-Dimethylthiomorpholino)sydnoniminium-hydrogensulfat

Zu einer Lösung von 305 g 4-Amino-3,3-dimethylthiomorpholin in 280 ml Wasser, die durch Zugabe von 37gew.%iger Schwefelsäure auf pH 6,2 gestellt wurde, tropft man innerhalb von 2,5 h bei 20°C und pH 6 - 6,5 Lösungen von 134 g Natriumcyanid in 440 ml Wasser und 209 g 39gew.-%igem Formaldehyd in 360 ml Wasser zu. Man rührt über Nacht bei Raumtemperatur, versetzt mit 1970 ml Essigsäureethylester und tropft bei 5 bis 10°C eine Lösung von 163 g Natriumnitrit in 260 ml Wasser zu. Dann stellt man mit 37gew.%iger Schwefelsäure auf pH 3,5, rührt 3 h bei 10°C nach und trennt bei Raumtemperatur die Phasen. Zu der organischen Phase, die das N-(3,3-Dimethylthiomorpholino)-N-nitrosoaminoacetonitril enthält, tropft man bei 20°C 268 g Monohydrat zu, rührt über Nacht und saugt bei 0°C den ausgefallenen Feststoff ab. Nach Waschen mit Essigsäureethylester und Trocknen im Vakuum erhält man 534 g (82 %) 3-(3,3-Dimethylthiomorpholino)sydnoniminium-hydrogensulfat.
Fp. 144°C (Zers.), Gehalt (HPLC): 100 %

### Vergleichsbeispiel 1:

### 3-(3,3-Dimethylthiomorpholino)sydnoniminium-chlorid

Zu einer Lösung von 146 g 4-Amino-3,3-dimethylthiomorpholin in 80 ml Wasser, die durch Zugabe von konz. Salzsäure auf pH 6,5 gestellt wurde, tropft man innerhalb von 2,5 h bei 20°C Lösungen von 61 g Natriumcyanid in 200 ml Wasser und 96 g 39gew.-%igem Formaldehyd in 165 ml Wasser zu, wobei der pH durch gelegentliche Zugabe von konz. Salzsäure zwischen 6 und 6,5 gehalten wird. Man rührt über Nacht bei Raumtemperatur, versetzt mit 900 ml Essigsäureethylester und tropft bei 5 bis 10°C eine Lösung von 74,5 g Natriumnitrit in 120 ml Wasser zu. Dann stellt man mit konz. Salzsäure auf pH 3,5, rührt 2 h bei 10°C und trennt bei Raumtemperatur die Phasen. Man verdünnt die organische Phase mit 300 ml Essigsäureethylester, leitet bei 0 bis 5°C 73 g Chlorwasserstoff ein und läßt über Nacht unter Rühren auf Raumtemperatur kommen. Man saugt den ausgefallenen Feststoff ab, wäscht mit Essigsäureethylester nach, trocknet im Vakuum und erhält 209 g (83 %) eines Rohprodukts, das laut ¹H-NMR etwa 5 % Ammoniumchlorid sowie mehrere weitere Verunreinigungen, von denen eine 3,3-Dimethylthiomorpholin ist, enthält. Nach Umkristallisation aus Isopropanol erhält man 156 g (62 %) reines 3-(3,3-Dimethylthiomorpholino)sydnoniminiumchlorid.
Fp: 172°C (Zers.)

### Beispiel 7:

### 3-(cis-2,6-Dimethylpiperidino)-N-(4-methoxybenzoyl)sydnonimin

Zu einer Suspension von 147 g Natriumhydrogencarbonat in 750 ml Essigsäureethylester tropft man bei 8 bis 10°C zunächst 92,3 g Anissäurechlorid und dann eine Lösung von 149 g 3-(cis-2,6-Dimethylpiperidino)sydnoniminium-hydrogensulfat in 500 ml Wasser zu. Man rührt 3 h bei 10°C, trennt die wäßrige Phase ab, wäscht die organische Phase mit einer Natriumhydrogencarbonatlösung und engt dann die organische Phase bis zur beginnenden Kristallisation ein. Man saugt bei 0°C ab, trocknet im Vakuum und erhält 152 g (92 %) 3-(cis-2,6-Dimethylpiperidino)-N-(4-methoxybenzoyl)sydnonimin.
Fp 129°C, Gehalt (HPLC): 99 %.

### Beispiel 8:

### N-Benzoyl-3-(cis-2,6-dimethylpiperidino)sydnonimin

Analog dem Beispiel 7 setzt man 58,8 g Natriumhydrogencarbonat, 300 ml Essigsäureethylester, 30,9 g Benzoylchlorid, 58,9 g 3-(cis-2,6-Dimethylpiperidino)sydnoniminium-hydrogensulfat und 200 ml Wasser bei 10°C um. Das nach dem Einengen der organischen Phase erhaltene Rohprodukt wird aus Essigsäureethylester umkristallisiert. Man erhält 45,5 g (76 %) N-Benzoyl-3-(cis-2,6-dimethylpiperidino)sydnonimin.
Fp. 135 bis 137°C

### Beispiel 9:

### 3-(cis-2,6-Dimethylpiperidino)-N-ethoxycarbonyl-sydnonimin

Analog dem Beispiel 8 erhält man aus 58,8 g Natriumhydrogencarbonat, 300 ml Essigsäureethylester, 23,9 g Chlorameisensäureethylester, 58,9 g 3-(cis-2,6-Dimethylpiperidino)sydnoniminium-hydrogensulfat und 200 ml Wasser 40,5 g (76 %) Produkt.

### Beispiel 10:

### 3-(3,3-Dimethylthiomorpholino)sydnoniminium-hydrogenmaleinat

Eine Lösung von 8,2 g Maleinsäure und 5,6 g Natriumhydroxid in 100 ml Wasser wird bei 0 bis 5°C langsam zugetropft zu einer Lösung von 20 g 3-(3,3-Dimethylthiomorpholino)sydnoniminium-hydrogensulfat in 60 ml Wasser. Nach einstündigem Nachrühren saugt man den ausgefallenen Feststoff ab, wäscht mit 25 ml Eiswasser und trocknet im Vakuum.
Ausbeute: 19,0 g (89 %), Fp. 149°C (Zers.).

## Patentansprüche

1. Verfahren zur Herstellung von Sydnoniminium-hydrogensulfaten der allgemeinen Formel I, 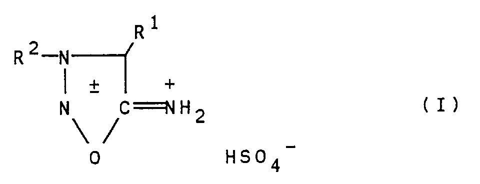 worin
R¹ Wasserstoff, Halogen, Alkyl, Cycloalkyl, Arylalkyl, Aryloxyalkyl, Arylthioalkyl, Alkoxyalkyl, Alkylthioalkyl oder Alkenylthioalkyl bedeutet;
R²- eine Aminogruppe der Formel 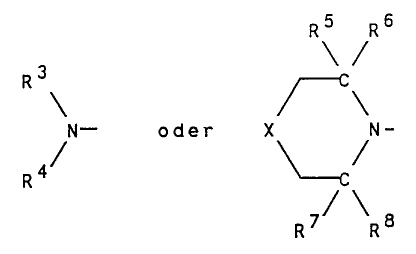 bedeutet;
R³ Alkyl, Alkenyl, Cycloalkyl, Bicycloalkyl, Tricycloalkyl, Alkoxyalkyl, Alkyl-S(O)ₙ-alkyl, Arylalkyl, das auch durch Alkyl oder Halogen substituiert sein kann, Phenyl, das auch durch Alkyl oder Halogen substituiert sein kann, Hydroxyalkyl, einen über eine Alkylenkette gebundenen heterocyclischen Ring oder einen Rest der Formel 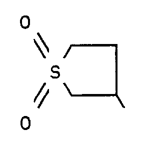 bedeutet;
R⁴ Wasserstoff bedeutet oder eine der Bedeutungen von R³ hat;
R⁵, R⁶, R⁷ und R⁸ unabhängig voneinander Wasserstoff oder Alkyl bedeuten;
X NR³, NSO₂R⁹, NCO₂Alkyl, S(O)ₙ, O, (CH₂)ₘ oder eine direkte Bindung bedeutet;
n 0, 1 oder 2 ist;
m 1, 2 oder 3 ist; und
R⁹ Alkyl, Aryl, Alkylaryl, Halogenaryl oder Dialkylamino bedeutet,
durch Umsetzung von N-Nitrosoaminoacetonitrilen der allgemeinen Formel II, 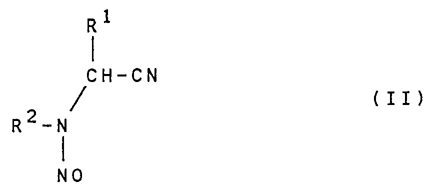 in der R¹ und R² die angegebenen Bedeutungen haben, mit Schwefelsäure in Gegenwart eines Lösungs- oder Dispergiermittels.

2. Verfahren nach Anspruch 1, dadurch gekennzeichnet, daß R¹ für Wasserstoff und R²- für eine Aminogruppe der Formel 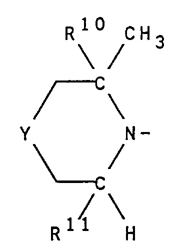 steht, in der
Y CH₂, O, S(O)n oder eine direkte Bindung bedeutet, n 0, 1 oder 2 ist und einer der Reste
R¹⁰ und R¹¹ eine Methylgruppe und der andere ein Wasserstoffatom bedeutet.

3. Verfahren nach Anspruch 1 und/oder 2, dadurch gekennzeichnet, daß R¹ für Wasserstoff und R² für den cis-2,6-Dimethylpiperidino-Rest steht.

4. Verfahren nach Anspruch 1 und/oder 2, dadurch gekennzeichnet, daß R¹ für Wasserstoff und R² für den 3,3-Dimethylthiomorpholino-Rest steht.

5. Verfahren nach einem oder mehreren der Ansprüche 1 bis 4, dadurch gekennzeichnet, daß bei der Umsetzung Ether oder Carbonsäurealkylester, bevorzugt Essigsäureethylester, als Lösungs- oder Dispergiermittel verwendet werden und die Umsetzung bei 0°C bis 50°C, bevorzugt bei 15°C bis 40°C, durchgeführt wird.

6. Verfahren nach einem oder mehreren der Ansprüche 1 bis 5, dadurch gekennzeichnet, daß pro Mol N-Nitrosoaminoacetonitril der allgemeinen Formel II 1 bis 2 mol Schwefelsäure, bevorzugt 1,1 bis 1,4 mol Schwefelsäure, eingesetzt werden und die Schwefelsäure einen Gehalt an H₂SO₄ von >95 Gew.-%, bevorzugt von >98 Gew.-% hat, besonders bevorzugt in Form von Monohydrat eingesetzt wird.

7. Sydnoniminium-hydrogensulfate der allgemeinen Formel I, in der R¹ und R² die in Anspruch 1 angegebenen Bedeutungen haben, in der aber, wenn R¹ für Wasserstoff steht, R² nicht für den cis-2,6-Dimethylpiperidino-Rest stehen kann.

8. 3-(3,3-Dimethylthiomorpholino)sydnoniminium-hydrogensulfat.

9. Verfahren zur Herstellung von N-Acylsydnoniminen der allgemeinen Formel III, 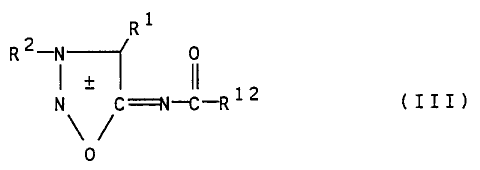 worin
R¹ und R² die in Anspruch 1 angegebenen Bedeutungen haben und
R¹² Aryl, durch 1 bis 3 Halogenatome und/oder 1 bis 3 Alkylreste und/oder 1 bis 3 Alkoxyreste und/oder 1 oder 2 Nitrogruppen mono-, di- oder trisubstituiertes Aryl, Arylalkenyl, Alkenylthioalkyl, Aryloxy, Alkoxycarbonyl, Pyridyl, Pyrrolyl, Imidazolyl, Pyrazolyl, Pyrazinyl, Pyrimidinyl, Pyridazinyl oder OR³ bedeutet oder eine der in Anspruch 1 angegebenen Bedeutungen von R³ hat, dadurch gekennzeichnet, daß ein Sydnoniminium-hydrogensulfat der allgemeinen Formel I mit einem Acylierungsmittel der allgemeinen Formel 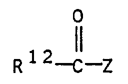 worin R¹² die angegebene Bedeutung hat und Z einen nucleophil abspaltbaren Rest bedeutet, umgesetzt wird.

10. Verfahren nach Anspruch 9, dadurch gekennzeichnet, daß R¹ Wasserstoff, R² den cis-2,6-Dimethylpiperidino-Rest, R¹² den 4-Methoxyphenyl-Rest und Z Chlor bedeutet.

11. Verfahren zur Herstellung von Sydnoniminium-salzen der allgemeinen Formel IV, 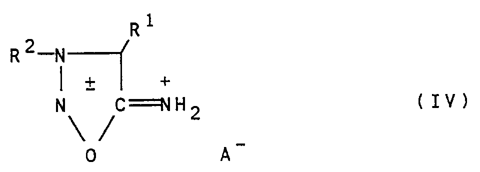 worin
R¹ und R² die in Anspruch 1 angegebenen Bedeutungen haben und
A⁻ das Anion einer pharmakologisch annehmbaren ein- oder mehrbasigen anorganischen oder organischen Säure HA ist, die nicht Schwefelsäure ist, wobei aber, wenn R¹ für Wasserstoff und R² für den cis-2,6-Dimethylpiperidino-Rest steht, HA nicht für Phosphorsäure oder Weinsäure stehen kann, dadurch gekennzeichnet, daß ein Sydnoniminium-hydrogensulfat der allgemeinen Formel I mit der Säure HA und/oder einem Salz hiervon umgesetzt wird.
